# EUROPÄISCHE PATENTSCHRIFT

(11) **EP 3 229 879 B1**
(45) Veröffentlichungstag und Bekanntmachung des Hinweises auf die Patenterteilung: **13.02.2019**
(21) Anmeldenummer: 15822907.0
(22) Anmeldetag: 09.12.2015
(51) Int. Cl.: A61M 15/00, A61M 15/08

(54) **INHALATIONSVORRICHTUNG, DEREN VERWENDUNG UND INHALATIONSSET**
INHALATION DEVICE, USE THEREOF, AND INHALATION KIT
DISPOSITIF D'INHALATION, SON UTILISATION ET TROUSSE D'INHALATION

(30) Priorität: 09.12.2014 DE 102014018085
(43) Veröffentlichungstag der Anmeldung: 18.10.2017
(73) Patentinhaber: Beller, Klaus-Dieter, 79341 Kenzingen (DE)
(72) Erfinder: Beller, Klaus-Dieter, 79341 Kenzingen (DE)
(74) Vertreter: mepat Patentanwälte
(86) Internationale Anmeldenummer: PCT/EP2015/002483
(87) Internationale Veröffentlichungsnummer: WO 2016/091386

(56) Entgegenhaltungen:
- US-A1- 2008 314 384
- US-A1- 2013 074 841
- US-A1- 2013 158 474

## Beschreibung

Die Erfindung betrifft eine Inhalationsvorrichtung, mit der eine inhalierbare Substanz, die in einem Behältnis in der Vorrichtung vorliegt, inhaliert werden kann. Ferner betrifft die Erfindung ein Inhalationsset, das aus Inhalationsvorrichtung und Behältnis besteht, sowie eine Verwendung der Inhalationsvorrichtung.

Bei herkömmlichen Inhalatoren, die mit einem Behältnis angewendet werden, das eine Substanz als Pulverdosis für eine Einzelanwendung ("Single-Dose") enthält, wie beispielsweise eine Kapsel oder ein Blister, ist eine feste Kombination aus Inhalator und Behältnis vorgegeben: Dort kann mit dem vorhandenen Inhalator nur ein bestimmter Behältnis-Typ verwendet werden. Bei Defekt oder Verlust des Inhalators kann der Anwender die noch vorhandenen Behältnisse nicht mehr verwenden.

Ein Beispiel für einen Single-Dose-Inhalator offenbart DE 10 2005 043 449 B3. Dort wird eine Inhalationsvorrichtung mit einem rohrförmigen Gehäuse beschrieben, das axial in zwei Gehäuseteile geteilt ist, die einen axialen Durchgangskanal begrenzen und von denen eines als Mund- oder Nasenstück und das andere als Lufteinlass ausgebildet ist. Zusammengefügt bilden beide Gehäuseteile eine Kammer zur Aufnahme einer Kapsel aus, die ein inhalierbares Pulver enthält. Zum Perforieren der in die Kammer eingelegten Kapsel weist jedes Gehäuseteil einen pyramidenförmig ausgebildeten, in die Kammer ragenden Dorn auf, sodass die Kapsel an gegenüberliegenden Enden perforiert und eine mit dem Durchgangskanal kommunizierende Passage durch die Kapsel erzeugt wird, wenn die Gehäuseteile axial aufeinander zu bewegt werden.

US 2013/0074841 A1 offenbart eine Inhalationsvorrichtung für in einer Blisterverpackung enthaltene Pulver. Diese Inhalationsvorrichtung ist mehrteilig und setzt sich aus einem Gehäuse mit einem Mundstück, das durch eine Verschlusskappe überdeckt werden kann, einem Zwischenboden und einem Deckel zusammen. Weiter wird die im Gehäuse zwischen dem Mundstück und einem abgestuften Gehäuseteil ausgebildete Wirbelkammer durch Deckplatten an Boden und Decke verschlossen. Im abgestuften Gehäuseteil werden durch Anordnung des Zwischenbodens Nebenluftkanäle ausgebildet. Auf dem Zwischenboden ist eine an die Kontur und Höhe eines aufzunehmenden Behälterteils der Blisterverpackung angepasste Aufnahme ausgebildet. Der Deckel ist schwenkbar an dem Gehäuse befestigt und weist schlitzförmige Lufteinlassöffnungen sowie Vertikalstege zur Begrenzung von Strömungswegen auf, von denen einer als Bogenbahn mit einem mindestens 180°-Krümmungsbereich gestaltet ist. In Schließstellung kommt der Deckel auf dem Zwischenboden zu liegen, sodass nach Entfernung der Behälterabdeckung Luftströme durch das Behälterteil der Blisterverpackung von beiden Enden geführt werden können, um nach Vereinigung in die Wirbelkammer zu gelangen.

Aus US 2013/158474 A1 ist eine Inhalationsvorrichtung zur nasalen Applikation eines Medikaments bekannt, wobei sich die Ausführungsformen der Inhalationsvorrichtung für unterschiedliche Medikamentenbehältnisse entsprechend unterscheiden. Eine Ausführungsform der Inhalationsvorrichtung für ein topfförmiges Dosierungsbehältnis besteht aus einem Körper mit einer Ausnehmung, in der das Behältnis aufgenommen werden kann. Am Ende der Ausnehmung ist ein Hohldorn angeordnet, von dem sich ein Auslasskanal durch den Körper zu einer Luftauslassöffnung erstreckt. Weiter wird in der Ausnehmung ein Kolben geführt, mit dem das Behältnis gegen den Hohldorn gedrückt und dadurch geöffnet werden kann. Der Kolben wird mittels eines Stempels an einem Schwenkarm betätigt, der an dem Körper auf einer Seite der Ausnehmung angelenkt ist. Auf der anderen Seite des Körpers erstreckt sich zur besseren Halterung und Bedienbarkeit ein Griffabschnitt, gegen den der Schwenkarm bewegt werden kann.

Ausgehend von diesem Stand der Technik ist es Aufgabe der vorliegenden Erfindung, eine einfach und kostengünstig herstellbare Inhalationsvorrichtung für Single-Dose-Behältnisse mit breitem Einsatzspektrum und einfacher Anwendung bereitzustellen.

Diese Aufgabe wird durch eine Inhalationsvorrichtung mit den Merkmalen des Anspruchs 1 gelöst.

Bevorzugte Ausführungsformen sind in den Unteransprüchen ausgeführt.

Ferner wird eine Verwendung der Inhalationsvorrichtung mit den Merkmalen des Anspruchs 12 offenbart.

Die weitere Aufgabe, ein einfach anwendbares und kostengünstig herstellbares Inhalationsset bereitzustellen, wird durch das Inhalationsset mit den Merkmalen des unabhängigen Anspruchs 13 gelöst.

Eine erfindungsgemäße Inhalationsvorrichtung weist wie herkömmliche Inhalationsvorrichtungen einen Lufteinlass, einen als Mund- oder Nasenstück ausgebildeten Luftauslass und eine Ausnehmung auf, die zur Aufnahme eines Behältnisses mit einer inhalierbaren Substanz ausgebildet ist. Die inhalierbare Substanz kann beispielsweise ein wirkstoffhaltiges Pulver oder eine verdampfbare Aromazubereitung sein. In der Inhalationsvorrichtung erstreckt sich ein Luftkanal von dem Lufteinlass durch die Ausnehmung, in die ein Hohldorn ragt oder mehrere Hohldorne ragen, zu dem Luftauslass. Je nach Anzahl der Hohldorne erstreckt sich von dem jeweiligen Hohldorn eine Luftzufuhrleitung zu dem Lufteinlass oder eine Luftauslassleitung zu dem Luftauslass. Erfindungsgemäß weist die Inhalationsvorrichtung ähnlich einer Zange oder Wäscheklammer zwei Schenkel mit jeweils einem Wirkende und einem Betätigungsende auf, wobei die Schenkel mittels eines Gelenks verbunden sind, das zwischen den Wirkenden und den Betätigungsenden liegt. Die zur Aufnahme des Behältnisses ausgebildete Ausnehmung wird zwischen den einander zugewandten Seiten beider Schenkel an den Wirkenden definiert, wobei der oder die Hohldorn(e) an einer der einander zugewandten Seiten beider Schenkel innerhalb der Ausnehmung angeordnet ist/sind. In Abhängigkeit der Anordnung der Hohldorne sind der Lufteinlass und der Luftauslass an einer von der Ausnehmung abgewandten Seite zumindest eines der Schenkel an dem Wirkende angeordnet. Die erfindungsgemäße Inhalationsvorrichtung kann vorteilhaft äußerst klein dimensioniert werden und eine Größe ähnlich der eines Taschenfeuerzeugs aufweisen. Außerdem weist eine erfindungsgemäße Inhalationsvorrichtung ein breites Einsatzspektrum auf, da verschiedene Behältnisse, beispielsweise sowohl Blister als auch Kapseln, verwendet werden können. Erfindungsgemäß können bei der Inhalationsvorrichtung die Wirkenden durch Betätigung der Schenkel an den Betätigungsenden geöffnet oder geschlossen werden und das Gelenk ist derart ausgebildet, dass eine Offenstellung der Wirkenden bei Zusammenführen der Betätigungsenden bereitgestellt wird.

Ferner kann eine erfindungsgemäße Inhalationsvorrichtung eine Rückstellvorrichtung aufweisen, die mit beiden Schenkeln in Wirkverbindung steht, um eine Geschlossenstellung der Wirkenden bereitzustellen, d. h., dass die durch die Rückstellvorrichtung bereitgestellte Rückstellkraft dem Öffnen der Wirkenden entgegenwirkt bzw. das Schließen der Wirkenden unterstützt.

Vorzugsweise kann das Gelenk die Rückstellvorrichtung aufweisen, sodass die Inhalationsvorrichtung aus möglichst wenigen Komponenten aufgebaut ist. Dabei kann eine Schenkelfeder gleichzeitig als Gelenk und Rückstellvorrichtung dienen oder diese Funktionen können in einer besonders bevorzugten Ausführungsform durch ein Filmscharnier übernommen werden, das - wenn die Inhalationsvorrichtung aus Kunststoff gefertigt ist, einstückig mit den Schenkeln verbunden sein kann.

Es kann aber auch eine von dem Gelenk separate Rückstellvorrichtung vorgesehen sein, bei der es sich beispielsweise um ein elastisches Band, das um die Wirkenden angeordnet ist, oder um eine Feder, bevorzugt Schraubenfeder, die an den Betätigungsenden zwischen den Schenkeln angeordnet ist, handeln kann.

Eine weitere Ausführungsform einer erfindungsgemäßen Inhalationsvorrichtung weist einen einzigen Hohldorn auf, der mit einer integrierten Bypassluftzuleitung ausgestattet ist. Eine andere Ausführungsform einer erfindungsgemäßen Inhalationsvorrichtung sieht zumindest zwei Hohldorne vor, von denen jeweils zumindest einer über die zumindest eine Luftzufuhrleitung dem Lufteinlass und über die zumindest eine Luftauslassleitung dem Luftauslass zugeordnet ist. Es können jedem Hohldorn mehrere Luftzufuhr- bzw. Luftauslassleitungen zugeordnet sein. Die zwei oder mehrere Hohldorne können an einem der Schenkel angeordnet sein, sodass sich Lufteinlass und Luftauslass auf der gleichen Seite an dem gleichen Schenkel befinden. Alternativ kann jeweils zumindest einer der zwei oder mehr Hohldorne an jedem Schenkel angeordnet sein, sodass Lufteinlass und Luftauslass an beiden Schenkeln in entgegengesetzte Richtungen weisen. Dabei können die Hohldorne wahlweise einander gegenüberliegend oder versetzt zueinander angeordnet sein.

Kostengünstig kann die Inhalationsvorrichtung teilweise oder vorzugsweise komplett und einstückig aus Kunststoff im Spritzguss gefertigt sein. Gegebenenfalls kann der oder können die Hohldorn(e) statt aus Kunststoff aus Metall sein und quasi als Einleger in die spritzgegossene Inhalationsvorrichtung integriert sein.

So kann die kostengünstig im Kunststoffspritzguss hergestellte Inhalationsvorrichtung vorteilhaft auch als Ersatzinhalator vorgesehen werden und eignet sich auch zum Vertrieb oder Verteilung in Ländern der Dritten Welt.

Jeder Hohldorn kann pyramidenförmig sein, wobei Pyramiden mit dreieckiger Grundfläche bevorzugt sein können, da hierbei die Kanten ein optimales Schneidergebnis beim Durchdringen der Behälterwand zeigen. Weist die Inhalationsvorrichtung mehr als einen Hohldorn auf, können diese auch unterschiedlich ausgeführt sein.

Zur Verbesserung der Luftströmungsbedingungen durch den Behälter kann der Lufteinlass und/oder die Luftzufuhrleitung aerodynamische Luftumleitungselemente aufweisen, die beispielsweise den Luftstrahl in Drehung bzw. Torsion versetzten. Diese Luftumleitungselemente sind bevorzugt einstückig mit der Inhalationsvorrichtung und nicht als separate Elemente geschaffen, insbesondere wenn die Inhalationsvorrichtung im Kunststoffspritzguss gefertigt ist.

Bei einem pyramidenförmigen Hohldorn kann auf jeder Seite jeweils eine Luftzufuhrleitung vorgesehen sein. Die Luftzufuhrleitungen können, um den angesaugten Luftstrom in Torsion zu versetzen, unsymmetrisch angeordnet sein, d. h. bei einer gleichmäßigen dreiseitigen Pyramide nicht im 120° Winkel. Alternativ oder zusätzlich können die Luftzufuhrleitungen unterschiedliche Durchmesser aufweisen, um am Einlass in den Behälter einen gewünschten Wirbel zu erzeugen.

Ferner kann die Inhalationsvorrichtung, insbesondere da sie auch zur Verwendung mit Behältnissen vorgesehen ist, die eine verdampfbare Substanz enthalten, eine Heizeinrichtung mit einem Heizelement umfassen, das in die Ausnehmung ragt oder in oder entlang dem Lufteinlass und/oder der Luftzufuhrleitung angeordnet ist. Eine solche Heizeinrichtung kann mit einer Energiequelle wie einer Batterie oder einem Akkumulator betrieben werden, für die/den in einem der Schenkel ein Aufnahmefach mit entsprechenden elektrischen Kontakten, die mit der Heizeinrichtung verbunden sind, vorgesehen sein kann.

Um die Desagglomeration des zu inhalierenden Pulvers zu verbessern, kann die Inhalationsvorrichtung einen Desagglomerationsaufsatz aufweisen, der lösbar oder unlösbar auf dem Luftauslass angeordnet ist und eine Desagglomerationsstruktur aufweist, die sich über den gesamten Querschnitt des Luftauslasses erstrecken kann.

Die Desagglomerationsstruktur kann gitterartig ausgebildet sein und so zusätzlich noch zum Auffangen von Folienbruchstücken dienen, wobei die gitterartige Desagglomerationsstruktur plan oder gewölbt ausgebildet sein kann. Die Wölbungsrichtung, die konkav oder konvex sein kann, wie auch die Maschengröße der gitterartigen Desagglomerationsstruktur können in Anhängigkeit der Beschaffenheit des mit der Inhalationsvorrichtung zu inhalierenden Pulvers gewählt werden.

Erfindungsgemäß kann diese Inhalationsvorrichtung zur Perforation eines Behältnisses mit einer inhalierbaren Substanz und zur Bereitstellung der Substanz zur Inhalation verwendet werden.

Ein erfindungsgemäßes Inhalationsset umfasst eine erfindungsgemäße Inhalationsvorrichtung und zumindest ein Behältnis mit einer inhalierbaren Substanz.

Das Behältnis kann eine Einengung aufweisen, die bei Anordnung des Behältnisses in der Ausnehmung der Inhalationsvorrichtung zwischen der Luftzufuhrleitung und der Luftauslassleitung oder entsprechend zwischen mehreren Luftzufuhr- und Luftauslassleitungen angeordnet sein und für eine Beschleunigung des Luftstroms und dadurch für eine verbesserte Pulvermitnahme und Desagglomeration sorgen.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich schematische Darstellungen von Ausführungsbeispielen der Erfindung.

Dabei zeigen:
- **Fig. 1**: eine schematische Seitenansicht einer erfindungsgemäßen Inhalationsvorrichtung a) mit angedeutetem Luftströmungskanal und b) in geöffnetem Zustand,
- **Fig. 2**: eine schematische Seitenansicht einer alternativen erfindungsgemäßen Inhalationsvorrichtung,
- **Fig. 3**: eine schematische Seitenansicht einer weiteren alternativen erfindungsgemäßen Inhalationsvorrichtung,
- **Fig. 4**: eine einstückig ausgeführte erfindungsgemäße Inhalationsvorrichtung in a) perspektivischer Ansicht, b) Längsschnittansicht, c) Draufsicht, d) Untersicht und e) Frontsicht,
- **Fig. 5**: ein zur Verwendung mit der Inhalationsvorrichtung aus Fig. 4 geeignetes Pulver- oder Aromabehältnis,
- **Fig. 6**: eine einstückig ausgeführte erfindungsgemäße Inhalationsvorrichtung mit alternativ gestalteter Aufnahmefläche/Kavität in a) perspektivischer Ansicht und b) Frontsicht,
- **Fig. 7**: ein zur Verwendung mit der Inhalationsvorrichtung aus Fig. 6 geeignetes Pulver- oder Aromabehältnis,
- **Fig. 8**: eine einstückig ausgeführte erfindungsgemäße Inhalationsvorrichtung mit einem einzigen Hohldorn in a) perspektivischer Ansicht, b) Längsschnittansicht,
- **Fig. 9**: ein zur Verwendung mit der Inhalationsvorrichtung aus Fig. 8 geeignetes Pulver- oder Aromabehältnis,
- **Fig. 10**: eine einstückig ausgeführte erfindungsgemäße Inhalationsvorrichtung mit zwei Hohldornen an einem Schenkel in a) perspektivischer Ansicht, b) Längsschnittansicht, c) Draufsicht und d) Untersicht,
- **Fig. 11**: ein zur Verwendung mit der Inhalationsvorrichtung aus Fig. 10 geeignetes Pulver- oder Aromabehältnis,
- **Fig. 12**: eine Seitenansicht einer einstückig ausgeführten Inhalationsvorrichtung mit zwei Hohldornen an einem Schenkel mit aufgenommenem Behältnis,
- **Fig. 13**: eine perspektivische Detailansicht der Inhalationsvorrichtung aus Fig. 12 mit aufgenommenem Behältnis,
- **Fig. 14**: a) bis d) unterschiedliche Hohldorne,
- **Fig. 15**: eine Seitenschnittansicht durch eine Inhalationsvorrichtung mit einer Desagglomerationsstruktur am Luftauslass,
- **Fig. 16**: eine Draufsicht auf die Inhalationsvorrichtung aus Fig. 15.

Die erfindungsgemäße Vorrichtung bezieht sich auf eine Inhalationsvorrichtung zur nasalen und/oder inhalativen Applikation gängiger Behältnisse wie Kapseln, Einzelblister oder Kartuschen/Cartridges, die eine inhalierbare Substanz enthalten. Dabei kann es sich um einen pulverförmigen oder verdampfenden oder verdampfbaren inhalierbaren Wirk- oder Aromastoff handeln.

Damit eine erfindungsgemäße Inhalationsvorrichtung als Ersatz- oder Notfallinhalator sowie zum Vertrieb oder zur Verteilung in Ländern der Dritten Welt, wo sich Patienten bzw. Hilfsorganisationen teurere Inhalatoren nicht leisten können, in Frage kommt, liegt ein Hauptaugenmerk auf der kostengünstigen Produktion, dem breiten Einsatzspektrum und der einfachen Handhabung. Daher ist dieser einfache Inhalator so ausgelegt, dass verschiedene Pulver- oder Aromabehältnisse - sowohl Blister als auch Kapseln etc. - damit verwendet werden können - es können allerdings auch Varianten der erfindungsgemäßen Inhalationsvorrichtung so ausgebildet sein, dass eine Kombination mit einem bestimmten Pulver- oder Aromabehältnis besonders geeignet ist.

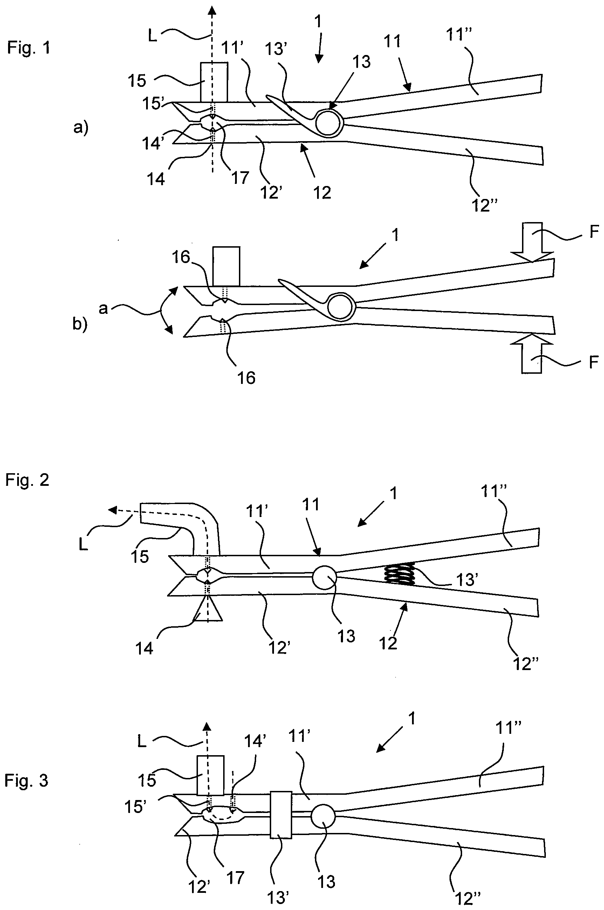

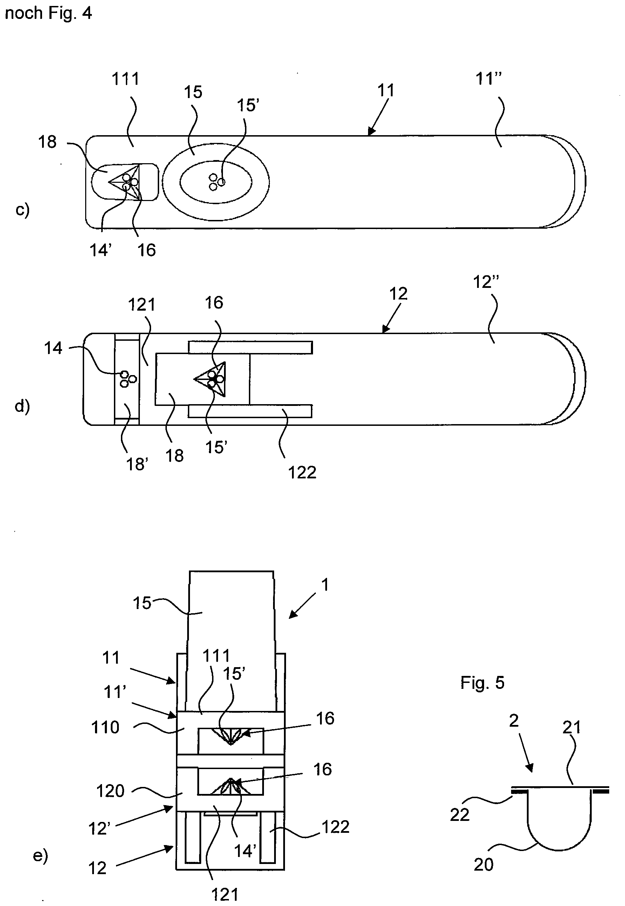

Eine erfindungsgemäße Inhalationsvorrichtung 1, wie in **Fig. 1 bis 4****,****6****,****8****,****10****,****12** gezeigt, ist ähnlich einer Zange aus zwei mittels eines Gelenks 13 verbundenen Schenkeln 11,12 aufgebaut. Die Schenkelenden auf der einen Seite des Gelenks 13 weisen als Wirkenden 11',12' Wirkstellen wie Nadeln, Dorne 16,16' oder Schneiden und eine Halte- bzw. Aufnahmevorrichtung 17 für ein Behältnis 2 (siehe **Fig. 5****,****7****,****9****,****12,13**) auf, während die Schenkelenden auf der anderen Seite des Gelenks 13 als Griffe bzw. Betätigungsenden 11",12" ausgebildet sind, um die Inhalationsvorrichtung 1 an den Wirkenden 11',12' zu öffnen bzw. zu schließen. Die Wirkenden 11',12' werden geöffnet, um ein Behältnis 2 einzulegen oder zu entfernen; beim Schließen der Wirkenden 11',12' wird das eingelegte Behältnis 2 durch das jeweilige Öffnungsmittel - Nadeln, (Hohl-)Dorne 16,16' oder Schneiden - geöffnet bzw. perforiert. Sind die Wirkenden 11',12' geschlossen, ist die Inhalationsvorrichtung 1 mit dem eingelegten Behältnis 2 verwendungsbereit.

Je nach Ausführung von Schenkeln und Gelenk kann die Betätigungsbewegung an den Betätigungsenden gleich- oder gegenläufig mit der Bewegung an den Wirkenden sein. Gleichläufig bedeutet, dass ein Zusammenführen der Betätigungsenden ein Schließen der Wirkenden und ein Auseinanderbewegen der Betätigungsenden ein Öffnen der Wirkenden bewirkt. Bei gegenläufiger Funktionsweise (wie in den Figuren gezeigt) bewirkt ein Zusammenführen der Betätigungsenden 11",12" ein Öffnen der Wirkenden 11',12' und umgekehrt. Generell kommen alle Gelenke in Frage, die auch als Zangengelenke bekannt sind: Ein einfaches aufgelegtes Gewerbe, bei dem die Schenkel übereinander gelegt und mit einem Gelenkbolzen verbunden sind, ein durchgestecktes Gewerbe, bei dem ein Schenkel durch eine Öffnung im anderen Schenkel hindurchgeführt ist oder eine gelenkig wirkende elastische Verbindung beider Schenkel.

Dabei kann vorgesehen sein, dass die Griffe bzw. Betätigungsenden als Hebelarme (Kraftarme) länger ausgebildet sind als die Wirkenden (Lastarme), sodass ein günstigeres Übersetzungsverhältnis zwischen der an den Betätigungsenden aufzubringenden Kraft und dem Öffnungsweg der Wirkenden erreicht werden kann.

Generell kann sowohl zum Öffnen als auch zum Schließen der Wirkenden eine Betätigung an den Betätigungsenden erforderlich sein, bevorzugt jedoch wird eine erfindungsgemäße Inhalationsvorrichtung eine Rückstellvorrichtung aufweisen, die eine Vorspannung für geschlossene Wirkenden bereitstellt. Wie in **Fig. 1b** skizziert, muss zum Überführen der Wirkenden 11',12' in Offenstellung (angedeutet durch Doppelpfeil a) einer solchen Inhalationsvorrichtung 1 diese Vorspannung durch Aufbringen der Kraft an den Betätigungsenden 11",12" (angedeutet durch die Blockpfeile F) überwunden werden; nach Einlegen des Behältnisses allerdings schließen die Wirkenden 11',12' selbsttätig (gehen in Geschlossenstellung), wenn keine Kraft mehr auf die Betätigungsenden ausgeübt wird, und versetzten so die Inhalationsvorrichtung 1 durch das dabei erfolgende Perforieren des Behältnisses in Verwendungsbereitschaft, wie in **Fig. 1a**, allerdings ohne Behältnis, dargestellt ist.

Der gestrichelte Pfeil L in **Fig. 1a, 2 und 3** deutet den Weg einer Luftströmung durch die Inhalationsvorrichtung 1 an, wenn in der Ausnehmung 17 ein Behältnis (nicht dargestellt) aufgenommen und durch die Hohldorne 16 perforiert ist.

Die Luftströmung L wird durch einen Einatemzug erzeugt, den ein Anwender an dem als Mundstück ausgeformten Luftauslass 15 vornimmt. Generell kann der Luftauslass 15, wie in **Fig. 2** angedeutet, auch als Nasenrüssel ausgeformt sein. Durch einen Lufteinlass 14, der wie in **Fig. 2** auch als Einlasstrichter oder ähnliches ausgeformt sein kann, strömt in der Anwendung der Inhalationsvorrichtung 1 Umgebungsluft durch eine Luftzufuhrleitung 14' zu einem ersten Hohldorn 16, der in die Ausnehmung 17, in der das Behältnis (nicht dargestellt) aufgenommen ist, hineinragt und dabei eine Wand dieses Behältnisses durchdringt. Die Luftströmung L passiert das Behältnis, reißt dabei die enthaltene Substanz, d. h. ein Pulver oder verdampfte Aromen, mit und tritt an dem zweiten Hohldorn 16 aus, von dem sich die Luftauslassleitung 15' zu dem Luftauslass 15 erstreckt. Der Lufteinlass 14 oder die Luftzufuhrleitung 14' kann aerodynamische Luftumleitungselemente (nicht in den Figuren dargestellt) enthalten, die den eingezogenen Luftstrahl in Drehung bzw. Torsion versetzten, wobei das oder die Luftumleitungselemente kein separates Bauteil, sondern einstückig mit dem entsprechenden Schenkel vorzugsweise im Spritzguss geschaffen sind.

Der Lufteinlass 14 und Luftauslass 15 können, wie in **Fig. 1 und 2** gezeigt, auf beide Schenkel 11,12 verteilt sein, oder, wie in **Fig. 3** dargestellt, an einem Schenkel 11 angeordnet sein. Eine mit der Anordnung des Lufteinlasses 14 und Luftauslasses 15 verbundene Anordnung der Hohldorne 16 (an einem Schenkel oder beiden, gegenüberliegend oder versetzt) kann sich auch nach einem gewünschten Strömungsverlauf durch das Behältnis richten. Ferner kann die Strömung durch die Form der Öffnungen in dem Hohldorn 16 sowie einer Kantenkontur dieser Öffnungen (scharfkantig, abgerundet etc.) beeinflusst werden.

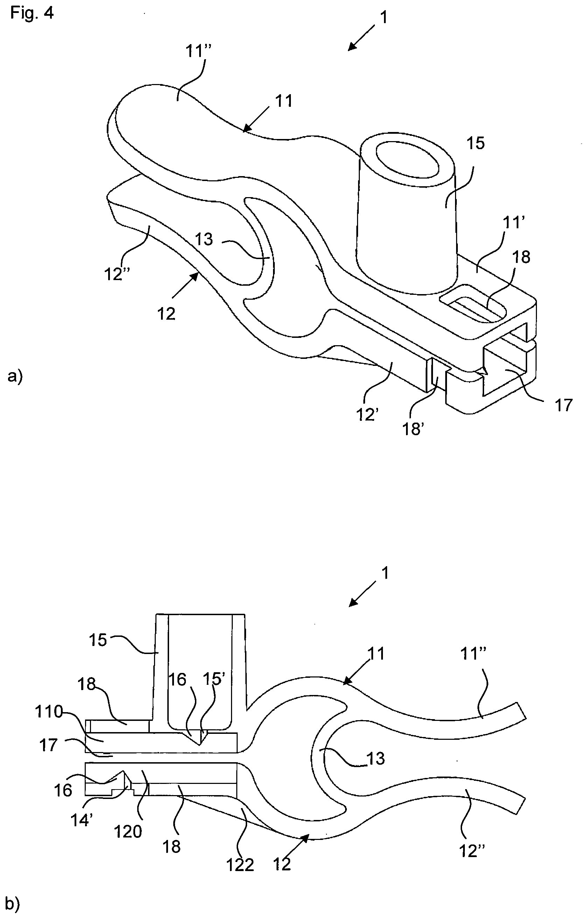

Vorteilhafterweise kann die Rückstellvorrichtung gleichzeitig als Gelenk ausgebildet sein, wie dies z. B. durch eine Schenkelfeder 13,13' (vgl. **Fig. 1**) wie bei einer Wäscheklammer oder durch das in den **Figuren 4****,****6****,****8****,****10** **und** **12** gezeigte Kunststofffilmgelenk 13, das die Schenkel 11,12 elastisch verbindet, der Fall ist.

Es ist aber nicht ausgeschlossen, dass auch eine von einem einfachen Gelenk 13 separate Rückstellvorrichtung 13' vorgesehen ist, wie in **Fig. 2** mit einer zwischen den Betätigungsenden 11",12" angeordneten Schraubenfeder und in **Fig. 3** mit einem um die Wirkenden 11',12' angeordneten elastischen Band gezeigt ist.

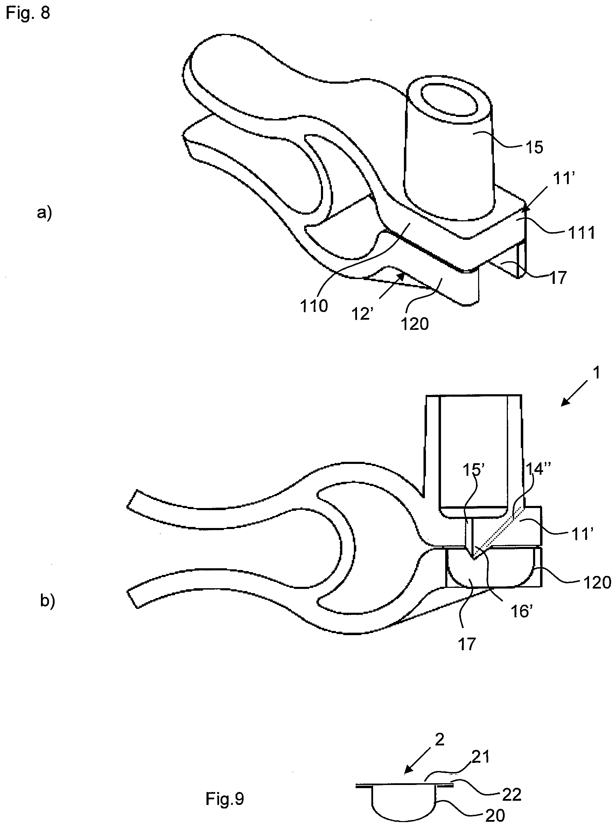

Die Wirkenden 11',12' einer erfindungsgemäßen Inhalationsvorrichtung 1 weisen Hohldorne 16 auf, die in die Ausnehmung 17 ragen und dem Lufteinlass 14 und Luftauslass 15 zugeordnet sind (bis auf eine in **Fig. 8** gezeigte Variante, in der lediglich ein dem Luftauslass zugeordneter Hohldorn 16' in die Ausnehmung 17 ragt, wobei allerdings eine Bypassleitung 14" als Lufteinlass integriert ist).

Die Ausnehmung 17 kann an einem oder beiden Wirkenden 11',12' ausgebildet sein und je nach Gestaltung der Seitenwände 110,120 sowie der Decke 111 und des Bodens 121 als Kammer mit nahezu geschlossenen Wänden oder auch nur als Einschub mit seitlicher Begrenzung bzw. Auflagefläche ausgebildet sein. Die Kammer bzw. die Auflagefläche kann entsprechend einer vorbestimmten Behältnisform z. B. Kapsel, Cartridge oder Blister vorgeformt sein, um dem Anwender das Einlegen zu erleichtern.

Mit einer erfindungsgemäßen Inhalationsvorrichtung können sowohl Kapseln, Blister als auch spezielle Gefäße geöffnet werden, die perforierbare Bereiche aufweisen. Ein Pulverbehältnis, das mit der erfindungsgemäßen Inhalationsvorrichtung verwendet werden kann, kann auch einen frei beweglichen Desagglomerator enthalten, der im inspirativen Luftstrom umhergewirbelt wird und für verbesserte Desagglomeration und Dispersion des Pulvers sorgt.

Wie in den Beispielen aus **Fig. 1 bis 3** zu sehen, kann eine erfindungsgemäße Inhalationsvorrichtung mehrteilig sein und aus den zwei Schenkeln 11,12, dem Gelenk 13 und gegebenenfalls der separaten Rückstellvorrichtung 13' bestehen. Die Hohldorne 16 können einstückig mit den Schenkeln 11,12 hergestellt sein, es ist aber auch denkbar, dass diese z. B. als Einleger im spritzgegossenen Schenkel vorliegen oder im Nachhinein eingebaut wurden. Während die Schenkel vorzugsweise kostengünstig aus Kunststoff gefertigt sind, können die Hohldorne - wenn sie nicht einstückig mit den Schenkeln gespritzt werden - auch aus einem anderen Material sein, das beispielsweise Metall, aber auch ein anderer (härterer) Kunststoff sein kann.

Bevorzugt jedoch kann eine erfindungsgemäße Inhalationsvorrichtung wie in **Fig. 4****,****6****,****8****,** **10****,****12** zu sehen, einteilig als zangenähnliche Klemmeinrichtung ausgeführt sein, die das Behältnis, das die inhalierbare Substanz enthält, mittels Hohldornen perforiert, wenn die Wirkenden durch die Rückstell- bzw. Schließkraft zusammengeführt werden.

**Figuren 4a****) bis e)** zeigen eine erfindungsgemäße Inhalationsvorrichtung 1 in verschiedenen Ansichten. Diese Inhalationsvorrichtung 1 ist einstückig ausgeführt und wird vorzugsweise im Kunststoffspritzguss hergestellt. Beide Schenkel 11,12 sind über eine Art Filmscharnier 13 gelenkig miteinander verbunden, das so geformt ist, dass die Schenkel 11,12 derart vorgespannt sind, dass die Wirkenden 11',12' geschlossen sind. An dem oberen Schenkel 11 ist an dem Wirkende 11' ein Mundstück als Luftauslass 15 angeformt, der mit der Luftauslassleitung 15' verbunden ist, die sich durch den Hohldorn 16 erstreckt, der in die Ausnehmung 17 ragend an dem oberen Schenkel 11 angeordnet ist. Der Hohldorn 16 für die Luftzufuhrleitung 14' an dem unteren Schenkel 12 ragt axial versetzt in die Ausnehmung 17, wie in **Fig. 4b****) bis d)** zu sehen ist. Im vorliegenden Beispiel liegen jeweils drei Luftzufuhrleitungen 14' und Luftauslassleitungen 15' vor, die sich jeweils von dem Luftein- bzw. -auslass 14,15 zu dem jeweiligen Hohldorn 16 erstrecken.

Um den Lufteinlass 14 ist ein abgesetzter Einschnitt 18' vorgesehen, um etwa ein unbeabsichtigtes Verschließen des Lufteinlasses 14 mit einem Finger zu verhindern.

Die Ausnehmung 17 an den Wirkenden 11',12' der Schenkel 11,12 wird durch die Seitenwände 110 und 120 sowie die Decke 111 und den Boden 121 begrenzt. Allerdings weisen sowohl die Decke 111 als auch der Boden 121 gegenüber zu dem jeweiligen Hohldorn 16 ein Fenster 18 auf, sodass von außen überprüft werden kann, ob ein Behältnis korrekt eingelegt ist, um von beiden Hohldornen 16 durchdrungen zu werden. Für eine ausreichende Stabilität des unteren Schenkels 12 sorgen Stege 122, die sich von dem nach außen gewölbten Abschnitt des Wirkendes 12' bis neben das Fenster 18 erstrecken.

Die Hohldorne 16 sind im vorliegenden Beispiel pyramidenförmig mit dreieckiger Grundfläche geformt, wie vor allem in **Fig. 4 c)** **und d)** zu sehen ist. Die drei Kanten der Pyramide sind als Schneidkanten scharfkantig ausgeführt, zu welchem Zweck die Seitenflächen gegebenenfalls hohlkehlig ausgeformt sein können. An jeder Seitenfläche der Pyramide befindet sich eine Öffnung, die jeweils mit der Luftzufuhr- oder -auslassleitung 14',15' kommuniziert.

Zum Einlegen eines Behältnisses, das als inhalierbare Substanz ein wirkstoffhaltiges Pulver oder eine Aromazubereitung enthält, beispielsweise in Form eines Blisters 2, wie in **Fig. 5** skizziert, der aus einem Schalenelement 20 bestehen kann, das mit einem Folienelement 21 am abkragenden Rand 22 versiegelt ist, werden die Betätigungsenden 11",12" der Inhalationsvorrichtung 1 zusammengedrückt, sodass sich die Wirkenden 11',12' öffnen. Der Blister 2 kann dann in die Ausnehmung 17 eingelegt werden, um beim Schließen der Wirkenden 11',12', wenn die auf die Betätigungsenden 11",12" ausgeübte Kraft verringert wird, von den Hohldornen 16 durchdrungen zu werden. Sind die Wirkenden 11',12' geschlossen, kann der Rand 22 des Blisters 2 zwischen den Seitenwänden 110,120 geklemmt sein und die Hohldorne 16 ragen ins Innere des Blisters 2.

Bei den Hohldornen mit Schneidkanten wird die Wandung des Blisters ohne Splitterbildung zerschnitten und aufgebogen. Da die Öffnungen zu den Luftzufuhr- und - auslassleitungen 14',15' in einer Hohlkehlung liegen, kann ein Luftstrom ungehindert die aufgebogenen Wandstücke passieren.

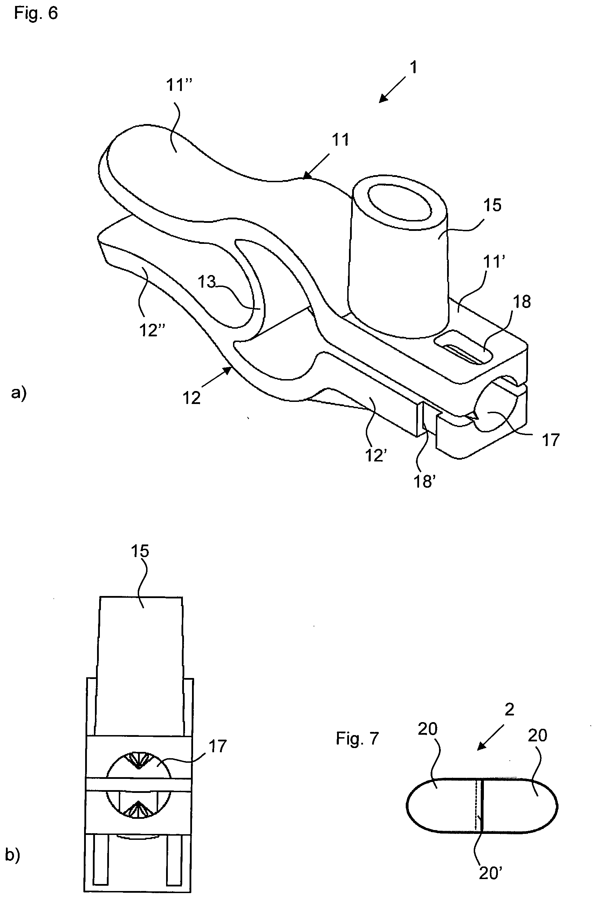

In den weiteren **Figuren 6****,****8****,****10****,****12** sind die Merkmale, die dem Ausführungsbeispiel aus **Fig. 4** entsprechen, nur noch teilweise mit Bezugszeichen versehen, da sich die Ausführungen im Wesentlichen bis auf die Gestaltung der Ausnehmung 17 zur Aufnahme unterschiedlicher Behältnisse und der Anordnung der Hohldorne 16 nicht oder nur unwesentlich unterscheiden, sodass die zum Beispiel im Zusammenhang mit **Fig. 4** gemachten Ausführungen auch für die weiteren Beispiele Gültigkeit haben.

Die Inhalationsvorrichtung in **Fig. 6a****) und b)** unterscheidet sich daher von dem in **Fig. 4** dargestellten Beispiel lediglich darin, dass die Ausnehmung 17 einen runden Querschnitt aufweist, und so zur Aufnahme eines zylindrischen Behältnisses wie einer Kapsel 2 (vgl. **Fig. 7**), die aus zwei an einem Überlapp 20' miteinander verbundenen Schalenelementen 20 besteht, optimiert ist.

Zwar werden hier in Bezug auf die **Figuren 4****,****6****,****8****,****10****,****12** Inhalationsvorrichtungen 1 beschrieben, deren Ausnehmungen 17 für bestimmte Behältnisse 2 optimiert sind; allerdings soll dies den erfindungsgemäßen Gegenstand nicht dahingehend einschränken, dass dieser nur mit bestimmten Behältnisse verwendet werden kann. So kann die Kapsel 2 aus **Fig. 5** durchaus auch in der für einen Blister optimierten Inhalationsvorrichtung 1 aus **Fig. 4** verwendet werden - gerade zu diesem Zweck sind die Fenster 18 hilfreich, mit denen die Positionierung des Behältnisses überprüft werden kann.

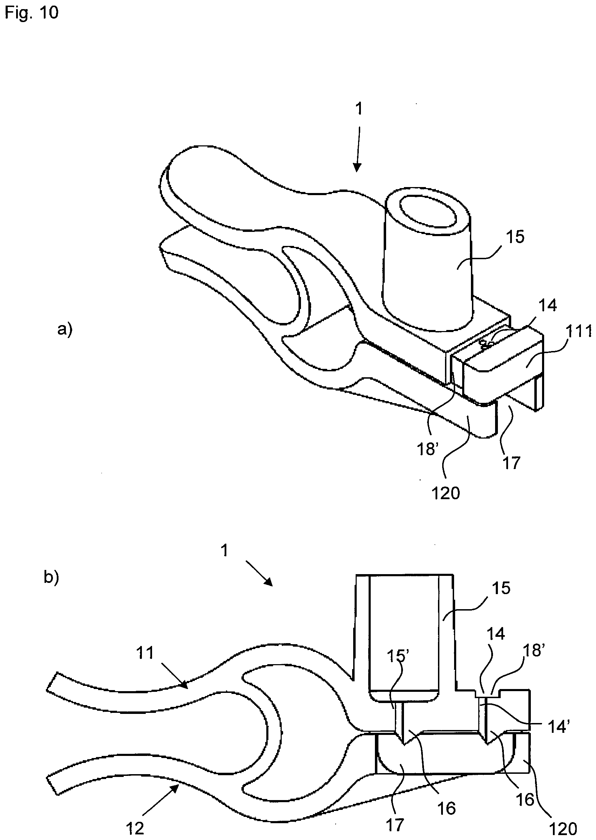

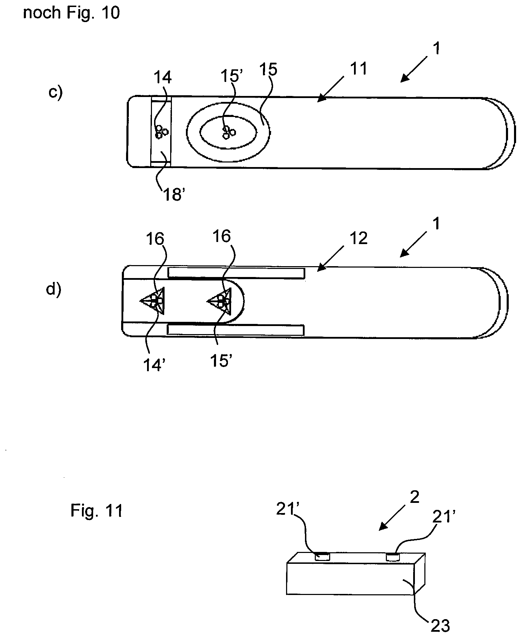

**Fig. 10** zeigt eine Inhalationsvorrichtung 1, bei der sowohl der Lufteinlass 14 als auch der Luftauslass 15 an dem oberen Schenkel 11 angeordnet sind. Entsprechend sind auch die Hohldörne 16 nur an dem oberen Schenkel 11 in die Ausnehmung 17 ragend angeordnet, während das Wirkende 12' des unteren Schenkels 12 mit Seitenwänden 120 ohne Boden ausgebildet ist. Die Seitenwände 120 weisen nach innen Anlageflächen auf, die entsprechend einem zur Aufnahme vorgesehenen Behältnis 2 geformt sein können. Das Wirkende 11' des oberen Schenkels 11 weist keine Seitenwände, sondern nur eine Decke 111 auf, durch die sich die Luftzufuhr- und -auslassleitungen 14',15' erstrecken. So bildet die Decke 111 des oberen Schenkels 11 einen "Deckel" für die von den Seitenwänden 120 begrenzte Ausnehmung 17. Ein Behältnis 2, das in einer Inhalationsvorrichtung 1 mit den Hohldornen 16 an einem Schenkel 11 verwendet werden kann, ist in **Fig. 11** mit einem quaderförmigen Spezialbehältnis 2 gezeigt. Entsprechend der Anordnung der Hohldorne 16 weist dieses Behältnis 2 zwei vorbestimmte Öffnungsstellen 21' für den Luftein- und -auslass auf. Diese Öffnungsstellen 21' in der Wand 23 des Behältnisses 2 sind mit einer Kunststoffschicht, bevorzugt einer Elastomerschicht (als "Dichtscheibe" oder Dichtring), überspritzt. Alternativ kann anstelle einer Öffnung auch eine Dünnstelle der Wand 23 an den Öffnungsstellen 21' vorgesehen sein. Vorteile dieser Ausführungsform liegen in der Dichtigkeit der Öffnungsstellen 21', die mit dünnerer Wandstärke ausgeführt sind, auch nach dem Zurückziehen der Hohldorne 16, sowie der Splitterfreiheit beim Öffnen und der durch die Elastomerschicht ermöglichten Führung der Hohldorne 16.

Ein solches Spezialbehältnis muss nicht quaderförmig sein, es kann auch anders geformt sein, z. B. abgerundet. So ist es z. B. denkbar, in der Inhalationsvorrichtung aus **Fig. 10** auch kapselförmige Behältnisse einzulegen, die dann an der ausgerundeten Anlagefläche der an der Seitenwand anliegen können.

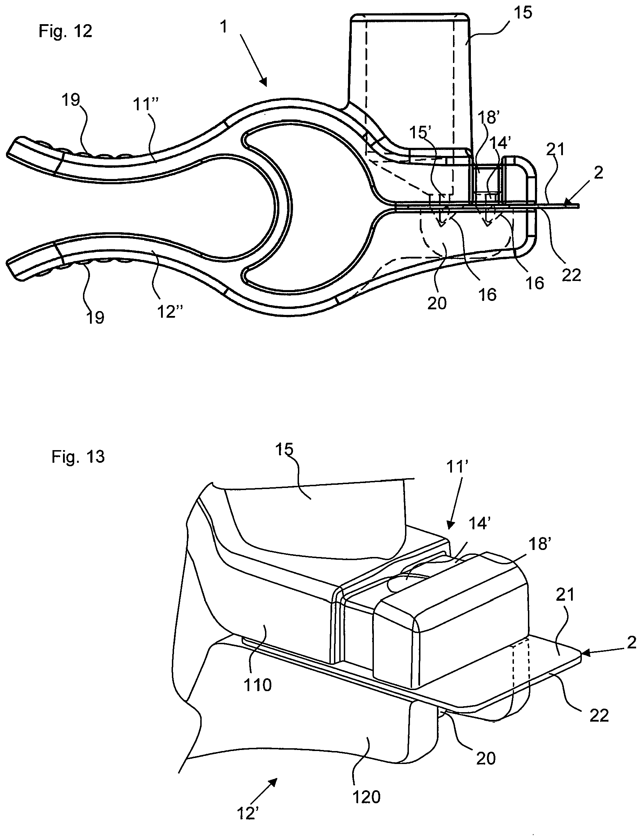

**Fig. 12 und 13** verdeutlichen einige Details oder alternative Ausführungen einer erfindungsgemäßen Inhalationsvorrichtung 1, die wie das Beispiel aus **Fig. 10** Luftein- und -auslass 14,15 an demselben Schenkel 11 aufweist und ebenfalls einstückig im Spritzguss hergestellt ist. Im Vergleich zu **Fig. 10** sind allerdings die Wirkenden 11',12' kürzer gestaltet und die Hohldorne 16 für Luftein- und -auslass 14,15 liegen näher beieinander, sodass ein kürzeres Behältnis 2 eingesetzt werden kann, das mit seinem Rand 22, an dem das Schalenelement 20 mit dem Folienelement 21 versiegelt ist, aus der Inhalationsvorrichtung 1 herausragt. Im Zuge der verkürzten Wirkenden 11',12' erstreckt sich der als Mundstück ausgeformte Luftauslass 15 bis zur Auswölbung des Schenkels 11 im Bereich des Gelenks 13. **Fig. 13** verdeutlich vor allem die Anordnung des blisterartigen Behältnisses 2 zwischen den Schenkeln 11,12. Der Rand 22 ragt, wie auch in **Fig. 12** zu sehen ist, nach vorne aus der Inhalationsvorrichtung heraus und ist zudem zwischen den Seitenwänden 110 und 120 eingeklemmt.

In **Fig. 12** ist zu sehen, dass die Betätigungenden 11",12" an den voneinander abgewanten Seiten mit einer Oberflächenstrukturierung 19 versehen sind, um dem Anwender die Betätigung zu erleichtern und einen sicheren Griff ohne Abrutschen zu ermöglichen. Eine solche Oberflächenstrukturierung 19 kann beispielsweise eine Riffelung oder Noppung sein.

Die Inhalationsvorrichtung 1 aus **Fig.** 8 ist als "Aroma-Inhalator" bzw. Vaporizer (Vaporisator) zum Inhalieren eines verdampenden oder verdampfbaren Wirk- oder Aromastoffes mit nur einem Hohldorn 16' konzipiert, der eine Luftauslassleitung 15' zum Luftauslass 15 aufweist. Diese Inhalationsvorrichtung 1 kann, muss aber nicht, einen Lufteinlass bzw. eine Bypassluftzuleitung 14" (gestrichelt dargestellt in **Fig. 8b**) aufweisen; denn der beim Einatmen in einem eingelegten Behältnis (siehe Blister 2 aus **Fig. 9**) erzeugte Unterdruck unterstützt/fördert das Verdampfen der enthaltenen Zubereitung. Wird allerdings der durch den Einatemsog beim Inhalieren in dem Behältnis erzeugte Unterdruck zu groß, kann der Anwender die Inhalationsvorrichtung 1 absetzen und Luft kann durch den Luftauslass in das Behältnis nachströmen, sodass erneut inhaliert werden kann. Vorzugsweise jedoch kann, um zu verhindern, dass ein in dem Behältnis beim Einatmen erzeugter Unterdruck zu groß wird, eine Bypasszuleitung 14" vorgesehen sein, damit beim Inhalieren Luft durch die Bypasszuleitung 14" in das Behältnis nachströmen kann. Eine Aromazubereitung kann eine Trägersubstanz umfassen, um das Verdampfen zu regulieren. Anders als in **Fig. 4** **und** **6** ist hier die Ausnehmung 17 nicht kammerartig ausgebildet; der untere Schenkel 12 weist am Wirkende 12' Seitenwände 120 mit geformten Anlageflächen, aber keinen Boden auf. Das Wirkende 11' des oberen Schenkels 11 besteht quasi nur aus der Decke 111, die einen "Deckel" zu den Seitenwänden 120 des unteren Schenkels 12 bildet. Gegebenenfalls kann auch hier vorgesehen sein, dass der Rand 22 des Blisters 2 zwischen der Decke 111 und den Seitenwänden 120 geklemmt wird.

Die mit der erfindungsgemäßen Inhalationsvorrichtung inhalierbaren Wirk- und Aromastoffe können sowohl als Pulver als auch als verdampfende oder verdampfbare feste (pastöse) oder flüssige Zubereitung vorliegen und umfassen neben den Aromastoffen (z. B. ätherische Öle) alle zur Inhalation bekannten Medikamente zur Behandlung von Atemwegserkrankungen und Schmerz, einschließlich Migräne, und psychischer Beeinträchtigungen wie Nervosität oder Depressionen, sowie zur Vaccination und zur Insulin-Therapie.

Eine erfindungsgemäße Inhalationsvorrichtung kann somit nicht nur mit Pulverbehältnissen angewendet werden, sodass die Inhalation eines wirkstoffhaltigen Pulvers als Aerosol ermöglicht wird, sondern auch mit Behältnissen, die eine verdampfbare Wirkstoff- oder Aromazubereitung enthalten, sodass ein Dampf inhaliert wird, der keine Partikel sondern feinste Tröpfchen enthält.

Verdampfer, oder auch Vaporizer oder Vaporisator genannte Vorrichtungen zum Verdampfen von Wirkstoffen sind bekannt. Anders als bei Dampfinhalatoren wird hierbei nicht eine Lösung sondern die Substanz direkt verdampft, gegebenenfalls in einer Zubereitung mit einer Trägersubstanz. Die Wirkstoff- oder Aromazubereitung kann in flüssiger oder fester bzw. pastöser Form vorliegen.

Auf dem Wege der Verdampfung können neben Tabak ("elektrische Zigarette") auch pharmazeutische Drogen wie Cannabis oder auch Pflanzen, bzw. Pflanzenteile oder Extrakte inhaliert werden. Beispiele für Pflanzen mit beruhigender Wirkung sind Baldrian (*Valeriana officinalis*) als Beruhigungsmittel und gegen krampfartige Beschwerden im Magen-Darmbereich, *Hopfen*(*Humulus lupulus*) als leichtes Einschlaf- und Beruhigungsmittel; Johanniskraut (*Hypericum perforatum*) wird zur Behandlung von leichten bis mittelstarken depressiven Verstimmungen oder nervöser Unruhe eingesetzt, Lavendel (*Lavandula angustifolia*) als Sedativum bei Unruhezuständen und Einschlafstörungen, aber auch bei nervösen Magen-Darm- und Gallenbeschwerden, und Passionsblume (*Passiflora incarnata*) gegen nervöse Unruhezustände, als Einschlafhilfe oder auch als angst- und krampflösendes Mittel. Auch Zitronenmelisse (*Melissa officinalis*) wirkt beruhigend und verdauungsfördernd.

Weitere Beispiele für Pflanzen mit medizinischen Wirkstoffen sind Damiana (*Turnera diffusa*), ein natürliches Aphrodisiakum und gegen Erkältungen Infektionskrankheiten oder Erkrankungen der Blutgefäße und als Tonikum einsetzbar, Eukalyptus (*Eucalyptus globulus*), der wegen schleimlösender, schwach krampflösender und antibakterieller Wirkung bei Erkältungskrankheiten und Asthma eingesetzt wird, Isländisches Moos (*Cetraria islandica*) gegen Schleimhautreizungen im Mund- und Rachenraum, sowie bei Entzündungen der Magen- und Darmschleimhaut. Zudem wirkt Isländisch Moos gegen Brechreiz, ist appetitsteigernd, belebend und kräftigend. Kamille (*Chamomilla recutita*) wird als Entzündungshemmer, zur Krampflösung, gegen Blähungen und als Magenmittel eingesetzt. Pfefferminze (*Mentha* × *piperita*) wirkt anregend auf Gallenfluss und Gallensaftproduktion, krampflösend bei Beschwerden im Magen-Darm-Bereich, antimikrobiell und antiviral. Ferner wird das ätherische Öl gegen Migräne, Kopf- und Nervenschmerzen sowie bei Erkältungskrankheiten verwendet; Salbei (*Salvia officinalis*) mit bakterien-, entzündungshemmender sowie adstringierender Wirkung, indiziert bei Entzündungen des Mund- und Rachenraumes. Schafgarbe (*Achillea spp*.) wird als Tonikum bei Verdauungsstörungen und Koliken eingesetzt, die ätherischen Öle wirken schleimlösend. Thymian (*Thymus vulgaris*) wird bei Infekten der oberen Luftwege, bei Bronchitis und Keuchhusten eingesetzt. Yohimbe (*Pausinystalia yohimbe*) wirkt als Aphrodisiakum und Potenzmittel.

Berauschende, euphorisierende oder sedierende, halluzinogene, psychoaktive oder gedächtnisöffnenden Wirkung haben neben Cannabis (*Cannabis sativa*) Afrikanisches Löwenohr (*Leonotis leonurus, "Wild Dagga"*), Ayahuasca, Yaje (*Banisteriopsis caapi*), Blauer Lotus (*Nymphaea* caerulea),Fliegenpilz (*Amanita muscaria*), Kratom (*Mitragyna specio*sa), Sinicuichi (*Heimia salicifolia*), Steppenraute (*Peganum harmala*) und Wahrsagesalbei (*Salvia divinorum*). Diese Pflanzen können auch weitere Inhaltsstoffe mit Arzneiwirkung, z. B. zur Schmerzlinderung haben, die sich durch Verdampfen medizinisch sinnvoll applizieren lassen, da so keine Verbrennungsprodukte erzeugt werden.

Verdampfer bzw. Vaporizer unterscheiden sich vor allem hinsichtlich der Zuführung der zur Verdampfung erforderlichen Verdampfungswärme. So kann entweder die zu verdampfende Substanz erwärmt werden oder die zugeführte Luft. Üblicherweise kann die Temperatur mittels geeigneter Regler entsprechend der gewünschten Verdampfungstemperatur eingestellt und konstant gehalten werden. Dabei wird die Substanz vorzugsweise nur soweit erwärmt, dass die gewünschten Inhaltsstoffe verdampfen.

Bei einem Verdampfer ohne Zuführung von Verdampfungswärme wird ein Luftstrom mit der gesättigten Luft um Verdampfer vermischt. Die Konzentration des Inhalats kann durch Aufteilung des Zuluftstroms in die Verdampferkammer und einen Bypasszweig gesteuert werden. Hierbei ist allerdings zu beachten, dass die Sättigungsmenge umgebungstemperaturabhängig ist.

Wird eine erfindungsgemäße Inhalationsvorrichtung als Verdampfer konzipiert, so kann eine Heizeinrichtung mit wenigstens einem Heizelement vorgesehen sein, um die zu verdampfende Substanz direkt oder den Zuluftstrom auf eine gewünschte Temperatur zu erhitzen. Die Heizeinrichtung kann neben dem Heizelement auch einen Energieträger wie einen Akku oder eine Batterie umfassen.

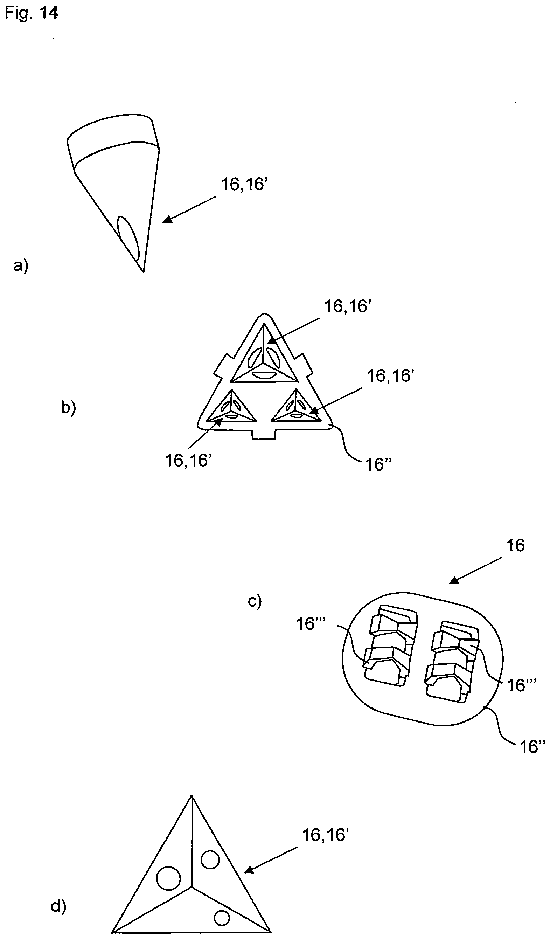

Schließlich soll darauf hingewiesen werden, dass die in der erfindungsgemäßen Inhalationsvorrichtung eingesetzten Hohldorne 16,16' nicht unbedingt als pyramidenförmiger Dorn mit dreieckiger Grundfläche ausgeführt sein müssen, wie in den Beispielen der **Figuren 4 und 10** zu entnehmen ist. Generell ist auch die Verwendung von scharfkantigen, spitzen Vierkant- oder Fünfkantdornen denkbar, bei denen dann allerdings zwangsläufig die Luftzufuhr- und -auslassöffnungen kleiner gestaltet sind. Auch können die Schneidkanten nicht so scharfkantig ausgeführt werden wie bei einem Dreikant. **Fig. 14** zeigt alternative Hohldornvorrichtungen 16, 16': In a) einen herkömmlichen kegelförmigen Hohldorn 16,16', mit dem allerdings die Behältniswand weniger durchschnitten als durchstochen wird; In b) eine Anordnung aus mehreren Hohldornen 16,16', die hier auf einer gemeinsamen Trägerstruktur 16" angeordnet sind (aber auch einstückig im Spritzguss mit einer Inhalationsvorrichtung gefertigt sein können), um dem Luftauslass 15 oder Lufteinlass 14 bzw. der Bypasszuluftleitung 14" zugeordnet zu werden, und in c) als Hohldorn 16,16' eine Platte 16" mit zwei Öffnungen, die jeweils von zwei Schneidstegen 16'" überbrückt werden. Diese Schneidstegen 16'" weisen geneigte Schnittkanten auf, um die Behälterwand zu durchschneiden, sodass ein Luftstrom durch die Öffnungen in den oder aus dem Behälter dringen kann. Wie bei der Trägerstruktur der Hohldornanordnung aus b) kann auch hier in einer Ausführung der Hohldornanordnung an einer Inhalationsvorrichtung die Platte an sich entfallen, sodass die von den Schneidstegen 16'" überbrückten Öffnungen direkt in dem jeweiligen Wirkende vorliegen.

Die Hohldorne können wie in der EP 1 762 265 A1 ausgeführt sein, auf deren Inhalt hiermit vollumfänglich Bezug genommen wird, und bei der auf jeder Seite des pyramidenförmigen Dorns je eine Lufteinlassöffnung vorgesehen ist. Vorzugsweise jedoch können bei einer erfindungsgemäßen Inhalationsvorrichtung die Luftzufuhröffnungen eines Hohldorns 16,16', wie in der Draufsicht in **Fig. 14d****)** zu sehen ist, nicht exakt symmetrisch - d. h. bei einem tetraedrischen Dorn im Winkel von 120° - orientiert sein. Ungleich verteilte Lufteinlassöffnungen insbesondere in Verbindung mit unterschiedlichen Durchmessern der Lufteinlassöffnungen - die Kanäle der Luftzufuhrleitungen 14' können z. B. der Reihe nach kleiner oder größer werden - sorgen dafür, dass die angesaugte Luft in eine Spiralbewegung versetzt wird, wenn sie in das Behältnis 2 eindringt, wodurch die Pulvermitnahme verbessert wird.

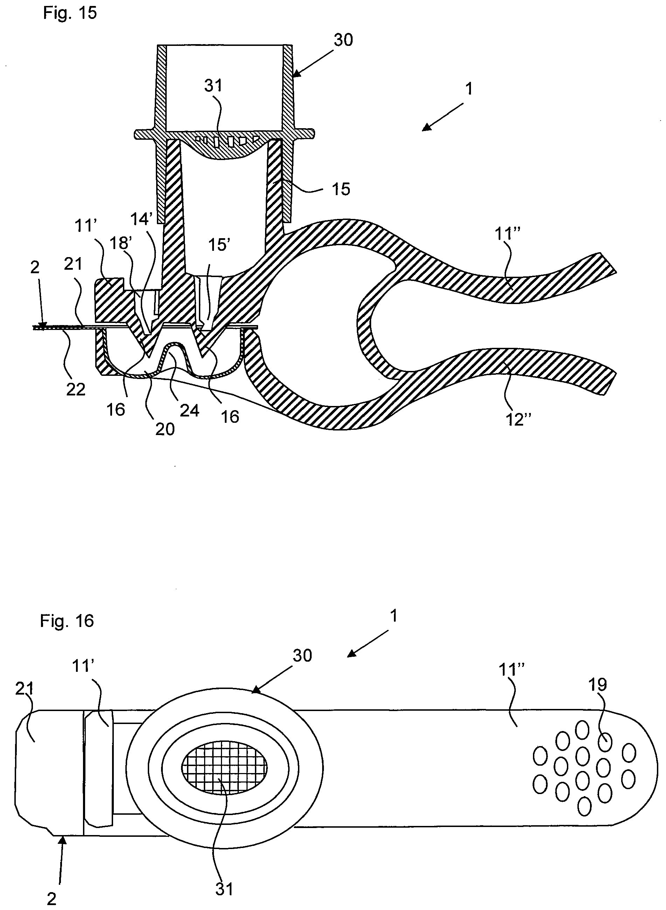

**Figuren 15 und 16** zeigen eine weitere Ausführungsform einer erfindungsgemäßen Inhalationsvorrichtung 1, bei der auf dem Luftauslass 15 ein Desagglomerationsaufsatz 30 aufgesetzt ist, der mit einer gitterartigen Desagglomerationsstruktur 31 ausgestattet ist. Diese Desagglomerationsstruktur 31 unterstützt die Desagglomeration der aus dem Behältnis mit dem Luftstrom mitgerissenen Pulverpartikel, dient aber auch als Fanggitter für Folienstücke, die gegebenenfalls beim Öffnen des Behältnisses entstehen können. Die gitterartigen Desagglomerationsstruktur 31 kann plan sein, vorzugsweise jedoch und in Abhängigkeit des zu inhalierenden Pulvers kann die gitterartigen Desagglomerationsstruktur 31 konkav oder konvex gewölbt ausgebildet sein. Die Wölbung und deren Richtung hat Einfluss auf die Desagglomeration und Deposition. Dabei bestimmt die Galenik des Pulvers, welche Form günstiger ist. Der Desagglomerationsaufsatz 30 kann fest auf dem Auslass 15 befestigt sein, er kann aber auch abnehmbar konzipiert sein, so dass sich die gitterartige Desagglomerationsstruktur 31 einfach reinigen lässt. Zudem kann ein abnehmbarer Desagglomerationsaufsatz einfach ausgetauscht werden, wenn ein anderes Pulver inhaliert werden soll, für das eine andere Form der gitterartigen Desagglomerationsstruktur 31 günstiger ist.

Weiter ist in **Fig. 15** ein alternativ ausgeführtes Behältnis 2 zu sehen, dessen Schalenelement 20 eine Einengung 24 aufweist, die bei Anordnung in der Inhalationsvorrichtung 1 zwischen dem Dorn 16 mit der Luftzufuhrleitung 14' und dem Dorn 16 mit der Luftauslassleitung 15' zu liegen kommt. Durch diese Einengung 24 wird der Luftstrom durch das Behältnis beschleunigt, wodurch die Pulvermitnahme und Desagglomeration unterstützt wird.

Jede erfindungsgemäße Inhalationsvorrichtung kann zumindest teilweise mit einer antiseptischen bzw. antibakteriellen und/oder antimikrobiellen Beschichtung versehen sein. So können besonders Lufteinlass, Luftzufuhrleitung, Hohldorne und Luftauslass beschichtet sein, um in der Anwendung der Inhalationsvorrichtung keine Keime zu inhalieren. Wegen der einfacheren Applikation kann aber auch die gesamte Inhalationsvorrichtung beschichtet sein. Ein Beispiel für eine solche Beschichtung ist Perlazid ® von Rilit, Endingen. Alternativ kann zur Herstellung der Inhalationsvorrichtung ein antiseptischer bzw. antibakterieller und/oder antimikrobieller Kunststoff verwendet werden.

Ferner kann der zur Herstellung einer erfindungsgemäßen Inhalationsvorrichtung eingesetzte Kunststoff einen Marker enthalten, der am fertigen Produkt nachgewiesen werden kann, um auf diese Weise einen Plagiatschutz zu erreichen, wie von der Firma Polysecure GmbH, Freiburg, angeboten wird, und beispielsweise in DE10 2008 060 675 B4, DE10 2012 017 710 A1, DE 10 2012 005 542 A1 oder DE 10 2012 003 519 A1 offenbart ist.

### BEZUGSZEICHENLISTE

- 1: Inhalationsvorrichtung
- 11,12: Schenkel
- 11', 12': Wirkende
- 11", 12": Betätigungsende
- 110,120: Seitenwand am Wirkende
- 111,121: Decke, Boden am Wirkende
- 122: Strebe
- 13, 13': Gelenk, Rückstellvorrichtung
- 14, 14', 14": Lufteinlass, Luftzufuhrleitung, Bypassluftzuleitung
- 15,15': Luftauslass, Luftauslassleitung
- 16, 16',: Hohldorn, Hohldorn mit integr. Bypassluftzuleitung,
- 16",16'": Trägerstruktur, Schneidstege
- 17: Ausnehmung zur Behältnisaufnahme
- 18, 18': Fenster, Einschnitt
- 19: Riffelung
- 2: Behältnis mit wirkstoffhaltigem Pulver oder Aromazubereitung
- 20, 20': Schalenelement, Überlapp
- 21, 21': Folienelement, Elastomerschicht
- 22: Siegelrand
- 23: Formgefäß
- 24: Einengung
- 30: Desagglomerationsaufsatz
- 31: Desagglomerationsstruktur, Gitter

## Patentansprüche

1. Inhalationsvorrichtung (1) mit einem Lufteinlass (14), einem als Mund- oder Nasenstück ausgebildeten Luftauslass (15) und einer Ausnehmung (17), die zur Aufnahme eines Behältnisses (2) mit einer inhalierbaren Substanz ausgebildet ist, wobei sich ein Luftkanal (L) von dem Lufteinlass (14) durch die Ausnehmung (17) zu dem Luftauslass (15) erstreckt und wobei die Inhalationsvorrichtung (1) zumindest einen in die Ausnehmung (17) ragenden Hohldorn (16,16') aufweist, von dem sich zumindest eine Luftzufuhrleitung (14',14") zu dem Lufteinlass (14) oder zumindest eine Luftauslassleitung (15') zu dem Luftauslass (15) erstreckt,
**dadurch gekennzeichnet, dass**
die Inhalationsvorrichtung (1) zwei Schenkel (11,12) mit jeweils einem Wirkende (11',12') und einem Betätigungsende (11",12") aufweist, wobei
- die Schenkel (11,12) mittels eines Gelenks (13) verbunden sind, das zwischen den Wirkenden (11',12') und den Betätigungsenden (11",12") liegt, und
- die Ausnehmung (17) zwischen den einander zugewandten Seiten beider Schenkel (11,12) an den Wirkenden (11',12') definiert wird, und
- derzumindest eine Hohldorn (16,16') an einer der einander zugewandten Seiten beider Schenkel (11,12) innerhalb der Ausnehmung (17) angeordnet ist,
- der Lufteinlass (14) und der Luftauslass (15) an einer von der Ausnehmung (17) abgewandten Seite zumindest eines der Schenkel (11,12) an dem Wirkende (11',12') angeordnet sind, und wobei die Wirkenden (11',12') durch Betätigung der Schenkel (11,12) an den Betätigungsenden (11",12") öffenbar oder schließbar sind, wobei das Gelenk (13) derart ausgebildet ist, dass eine Offenstellung (a) der Wirkenden (11',12') bei Zusammenführen der Betätigungsenden (11",12") bereitgestellt wird.

2. Inhalationsvorrichtung (1) nach Anspruch 1,
**dadurch gekennzeichnet, dass**
eine Rückstellvorrichtung (13') mit beiden Schenkeln (11,12) zur Bereitstellung einer Geschlossenstellung der Wirkenden (11',12') in Wirkverbindung steht.

3. Inhalationsvorrichtung (1) nach Anspruch 2,
**dadurch gekennzeichnet, dass**
das Gelenk (13) die Rückstellvorrichtung (13') aufweist und als Schenkelfeder oder bevorzugt als filmscharnierartiges Gelenk (13) ausgebildet ist,
oder dass die Rückstellvorrichtung (13')
- ein um die Wirkenden (11',12') angeordnetes elastisches Band oder
- eine zwischen den Betätigungsenden (11",12") angeordnete Feder (13'), bevorzugt eine Schraubenfeder, ist.

4. Inhalationsvorrichtung (1) nach zumindest einem der Ansprüche 1 bis 3,
**dadurch gekennzeichnet, dass**
die Inhalationsvorrichtung (1)
- einen einzigen Hohldorn (16') mit integrierter Bypassluftzuleitung (14") aufweist, oder
- zumindest zwei Hohldorne (16) aufweist, von denen jeweils zumindest einer über die zumindest eine Lufteinlassleitung (14') dem Lufteinlass (14) und über die zumindest eine Luftauslassleitung (15') dem Luftauslass (15) zugeordnet ist, wobei die zumindest zwei Hohldorne (16) beide an einem der Schenkel (11,12) angeordnet sind oder jeweils zumindest einer der zumindest zwei Hohldorne (16) an jedem Schenkel (11,12) angeordnet ist,
und wobei die Hohldorne (16) einander gegenüberliegend oder versetzt angeordnet sind.

5. Inhalationsvorrichtung (1) nach zumindest einem der Ansprüche 1 bis 4,
**dadurch gekennzeichnet, dass**
die Inhalationsvorrichtung (1)
- zumindest teilweise aus Kunststoff
- bevorzugt bis auf den zumindest einen Hohldorn (16,16') aus Kunststoff,
- besonders bevorzugt einstückig und komplett aus Kunststoff, bevorzugt im Spritzguss gefertigt ist.

6. Inhalationsvorrichtung (1) nach zumindest einem der Ansprüche 1 bis 5,
**dadurch gekennzeichnet, dass**
der zumindest eine Hohldorn (16,16') pyramidenförmig ist, vorzugsweise mit dreieckiger Grundfläche.

7. Inhalationsvorrichtung (1) nach zumindest einem der Ansprüche 1 bis 6,
**dadurch gekennzeichnet, dass**
der Lufteinlass (14) und/oder die Luftzufuhrleitung (14') aerodynamische Luftumleitungselemente aufweist, die bevorzugt einstückig mit der Inhalationsvorrichtung geschaffen sind.

8. Inhalationsvorrichtung (1) nach Anspruch 6 oder 7,
**dadurch gekennzeichnet, dass**
auf jeder Seite des pyramidenförmigen Hohldorns (16,16') jeweils eine Luftzufuhrleitung (14') vorgesehen ist, die vorzugsweise in Bezug auf den Hohldorn (16,16') unsymmetrisch angeordnet sind und/oder unterschiedliche Durchmesser aufweisen.

9. Inhalationsvorrichtung (1) nach zumindest einem der Ansprüche 1 bis 8,
**dadurch gekennzeichnet, dass**
die Inhalationsvorrichtung (1) eine Heizeinrichtung mit einem Heizelement umfasst, das - in die Ausnehmung (17) ragt, oder
- in oder entlang dem Lufteinlass (14) und/oder der Luftzufuhrleitung (14',14") angeordnet ist.

10. Inhalationsvorrichtung (1) nach zumindest einem der Ansprüche 1 bis 9,
**dadurch gekennzeichnet, dass**
die Inhalationsvorrichtung (1) einen Desagglomerationsaufsatz (30) aufweist, der lösbar oder unlösbar auf dem Luftauslass (15) angeordnet ist und eine Desagglomerationsstruktur (31) aufweist, die sich über den gesamten Querschnitt des Luftauslasses (15) erstreckt.

11. Inhalationsvorrichtung (1) nach Anspruch 10,
**dadurch gekennzeichnet, dass**
die Desagglomerationsstruktur (31) gitterartig ausgebildet ist, wobei die gitterartige Desagglomerationsstruktur (31) plan oder gewölbt ausgebildet ist, wobei eine Wölbungsrichtung und/oder eine Maschengröße in Abhängigkeit des mit der Inhalationsvorrichtung (1) zu inhalierenden Pulvers wählbar ist.

12. Verwendung einer Inhalationsvorrichtung (1) nach zumindest einem der Ansprüche 1 bis 11 zur Perforation eines Behältnisses (2) mit einer inhalierbaren Substanz und zur Bereitstellung der Substanz zur Inhalation.

13. Inhalationsset, umfassend eine Inhalationsvorrichtung (1) und zumindest ein Behältnis (2) mit einer inhalierbaren Substanz,
**dadurch gekennzeichnet, dass**
die Inhalationsvorrichtung eine Inhalationsvorrichtung (1) nach zumindest einem der Ansprüche 1 bis 11 ist.

14. Inhalationsset nach Anspruch 13,
**dadurch gekennzeichnet, dass**
das Behältnis (2) eine Einengung (24) aufweist, die bei Anordnung des Behältnisses (2) in der Ausnehmung (17) der Inhalationsvorrichtung (1) zwischen der zumindest einen Luftzufuhrleitung (14',14") und der zumindest einen Luftauslassleitung (15') angeordnet ist.

## Claims

1. Inhalation device (1) with an air inlet (14), an air outlet (15) designed in the form of a mouth piece or nose piece, and a recess (17) designed for accommodation of a container (2) with an inhalable substance, whereby an air channel (L) extends from the air inlet (14) through the recess (17) to the air outlet (15), and whereby the inhalation device (1) comprises at least one hollow mandrel (16, 16') that projects into the recess (17) and from which at least one air feed line (14', 14") extends to the air inlet (14) or at least one air outlet line (15') extends to the air outlet (15),
**characterised in that**
the inhalation device (1) comprises two legs (11, 12), with one acting end (11', 12') and one actuation end (11",12") each, whereby
- the legs (11, 12) are connected by means of a joint (13) that is situated between the acting ends (11', 12') and the actuation ends (11", 12"), and
- the recess (17) is being defined between the sides of the two legs (11, 12) on the acting ends (11', 12') that face each other, and
- the at least one hollow mandrel (16, 16') is arranged on one of the sides of the two legs (11, 12) that face each other within the recess (17),
- the air inlet (14) and the air outlet (15) are arranged on the acting end (11', 12') of at least one of the legs (11, 12) on a side facing away from the recess (17), and whereby the acting ends (11', 12') can be opened or closed by actuating the legs (11, 12) on the actuation ends (11",12"), whereby the joint (13) is designed appropriately such that an open position (a) of the acting ends (11', 12') is provided when the actuation ends (11", 12") are brought together.

2. Inhalation device (1) according to claim 1,
**characterised in that**
a restoring device (13') is in operative connection with both legs (11, 12) in order to provide a closed position of the acting ends (11', 12').

3. Inhalation device (1) according to claim 2,
**characterised in that**
the joint (13) comprises the restoring device (13') and is designed as a leg spring or preferably as a film hinge-like joint (13),
or **in that** the restoring device (13')
- is an elastic band that is arranged about the acting ends (11', 12') or
- is a spring (13') that is arranged between the actuation ends (11",12"), preferably a coil spring.

4. Inhalation device (1) according to at least one of the claims 1 to 3,
**characterised in that**
the inhalation device (1)
- comprises a single hollow mandrel (16') with integrated bypass air feed line (14"), or
- comprises at least two hollow mandrels (16) of which at least one is assigned to the air inlet (14) via the at least one air inlet line (14') and is assigned to the air outlet (15) via the at least one air outlet line (15'), whereby the at least two hollow mandrels (16) both are arranged on one of the legs (11, 12) or at least one of the at least two hollow mandrels (16) is arranged on each leg (11, 12),
and whereby the hollow mandrels (16) are arranged opposite from each other or offset from each other.

5. Inhalation device (1) according to at least one of the claims 1 to 4,
**characterised in that**
the inhalation device (1)
- is made from plastics, at least in part,
- is preferably made from plastics except for the at least one hollow mandrel (16, 16'),
- is particularly preferably made as a single part and completely of plastics, preferably by injection moulding.

6. Inhalation device (1) according to at least one of the claims 1 to 5,
**characterised in that**
the at least one hollow mandrel (16, 16') is pyramid-shaped, preferably with a triangular base.

7. Inhalation device (1) according to at least one of the claims 1 to 6,
**characterised in that**
the air inlet (14) and/or the air feed line (14') comprise aerodynamic air deflecting elements, which preferably are created such as to be the same part as the inhalation device.

8. Inhalation device (1) according to claim 6 or 7,
**characterised in that**
an air feed line (14') each is provided on each side of the pyramid-shaped hollow mandrel (16, 16'), which preferably are arranged asymmetrically with respect to the hollow mandrel (16, 16') and/or have different diameters.

9. Inhalation device (1) according to at least one of the claims 1 to 8,
**characterised in that**
the inhalation device (1) comprises a heating facility with a heating element that
- projects into the recess (17), or
- is arranged in or along the air inlet (14) and/or the air feed line (14', 14").

10. Inhalation device (1) according to at least one of the claims 1 to 9,
**characterised in that**
the inhalation device (1) comprises a deagglomeration attachment (30) that is arranged on the air outlet (15) such as to be detachable or non-detachable and comprises a deagglomeration structure (31) that extends across the entire cross-section of the air outlet (15).

11. Inhalation device (1) according to claim 10,
**characterised in that**
the deagglomeration structure (31) is provided to be mesh-like, whereby the mesh-like deagglomeration structure (31) is provided to be planar or curved, whereby a direction of curvature and/or a mesh size can be selected as a function of the powder to be inhaled with the inhalation device (1).

12. Use of an inhalation device (1) according to at least one of the claims 1 to 11 for perforation of a container (2) with an inhalable substance and for providing the substance for inhalation.

13. Inhalation set, comprising an inhalation device (1) and at least one container (2) with an inhalable substance,
**characterised in that**
the inhalation device is an inhalation device (1) according to at least one of the claims 1 to 11.

14. Inhalation set according to claim 13,
**characterised in that**
the container (2) comprises a constriction (24), which is arranged between the at least one air feed line (14', 14") and the at least one air outlet line (15') when is arranged the container (2) in the recess (17) of the inhalation device (1).

## Revendications

1. Dispositif d'inhalation (1) avec une entrée d'air (14), une sortie d'air (15) sous forme d'embout buccal ou d'embout nasal et une cavité (17) qui est formée pour la réception d'un récipient (2) contenant une substance inhalable, un conduit d'air (L) s'étendant de l'entrée d'air (14) vers la sortie d'air (15) par la cavité (17) et le dispositif d'inhalation (1) présentant au moins un mandrin (16, 16') en saillie dans la cavité (17), à partir duquel s'étend au moins un conduit d'amenée d'air (14', 14") vers l'entrée d'air (14) ou au moins un conduit d'échappement d'air (15') vers la sortie d'air (15), **caractérisé par le fait que**
le dispositif d'inhalation (1) présente deux côtés (11, 12), chacun avec une extrémité d'action (11',12') et une extrémité d'actionnement (11",12"),
- les côtés (11, 12) étant reliés par une charnière (13) qui se trouve entre les extrémités d'action (11',12') et les extrémités d'actionnement (11",12") et
- la cavité (17) étant définie entre les faces opposées des deux côtés (11, 12) aux extrémités d'action (11', 12') et
- au moins un mandrin (16, 16') étant disposé sur l'une des faces opposées des deux côtés (11, 12) à l'intérieur de la cavité (17),
- l'entrée d'air (14) et la sortie d'air (15) étant disposées sur l'une des faces au moins de l'un des côtés (11, 12), orientées à l'opposé de la cavité (17), à l'extrémité d'action (11', 12') et les extrémités d'action (11', 12') pouvant être ouvertes ou fermées par l'actionnement des côtés (11, 12) aux extrémités d'actionnement (11",12"),
la charnière (13) étant formée de manière à ce qu'une position ouverte (a) des extrémités d'action (11',12') soit mise à disposition lors de l'interaction des extrémités d'actionnement (11",12").

2. Dispositif d'inhalation (1) conformément à la revendication n°1,
**caractérisé par le fait que**
le dispositif de rappel (13') est en liaison active avec les deux côtés (11, 12) pour la mise en disposition d'une position fermée des extrémités d'action (11', 12').

3. Dispositif d'inhalation (1) conformément à la revendication n°2,
**caractérisé par le fait que**
la charnière (13) présente le dispositif de rappel (13') et est réalisée sous forme de ressort de torsion ou de préférence sous forme de charnière formant charnière-film (13)
ou que le dispositif de rappel (13')
- est un ruban élastique disposé autour des extrémités d'action (11', 12') ou
- est un ressort (13') disposé entre les extrémités d'actionnement (11",12"), de préférence un ressort hélicoïdal.

4. Dispositif d'inhalation (1) conformément au moins à l'une des revendications n°1 à n°3,
**caractérisé par le fait que**
le dispositif d'inhalation (1)
présente un mandrin (16') unique avec conduit d'air de dérivation (14") intégré ou
- présente au moins deux mandrins (16), desquels au moins un est affecté à l'entrée d'air (14) par au moins un conduit d'amenée d'air (14') et au moins un est affecté à la sortie d'air (15) par au moins un conduit d'échappement d'air (15'), au moins deux mandrins (16) étant les deux disposés sur l'un des côtés (11, 12) ou au moins l'un d'au moins deux mandrins (16) étant disposé sur chaque côté (11, 12)
et les mandrins (16) étant disposés en face ou en décalage l'un par rapport à l'autre.

5. Dispositif d'inhalation (1) conformément au moins à l'une des revendications n°1 à n°4,
**caractérisé par le fait que**
le dispositif d'inhalation (1)
- est au moins partiellement en matière synthétique
- de préférence sauf au moins un mandrin (16, 16'), en matière synthétique
- en particulier de préférence en une pièce et entièrement en matière synthétique, en étant de préférence réalisé dans le procédé de moulage par injection.

6. Dispositif d'inhalation (1) conformément au moins à l'une des revendications n°1 à n°5,
**caractérisé par le fait que**
au moins un mandrin (16, 16') est en forme pyramidale, de préférence avec une surface de base triangulaire.

7. Dispositif d'inhalation (1) conformément au moins à l'une des revendications n°1 à n°6,
**caractérisé par le fait que**
l'entrée d'air (14) et/ou le conduit d'amenée d'air (14') présente(nt) des éléments de redirection d'air aérodynamiques qui sont créés de préférence en une pièce avec le dispositif d'inhalation.

8. Dispositif d'inhalation (1) conformément à la revendication n°6 ou n°7,
**caractérisé par le fait que,**
de chaque côté du mandrin (16, 16') en forme pyramidale sont prévus des conduits d'amenée d'air (14') respectifs qui sont disposés de préférence de façon non symétrique par rapport au mandrin (16, 16') et/ou qui présentent des diamètres différents.

9. Dispositif d'inhalation (1) conformément au moins à l'une des revendications n°1 à n°8,
**caractérisé par le fait que**
le dispositif d'inhalation (1) comprend un dispositif de chauffage avec un élément chauffant qui - est en saillie dans la cavité (17) ou
- qui est disposé dans ou le long de l'entrée d'air (14) et/ou du conduit d'amenée d'air (14', 14").

10. Dispositif d'inhalation (1) conformément au moins à l'une des revendications n°1 à n°9,
**caractérisé par le fait que**
le dispositif d'inhalation (1) présente un embout de désagglomération (30) qui est disposé de façon amovible ou non amovible sur la sortie d'air (15) et qui présente une structure de désagglomération (31) qui s'étend au-dessus de toute la section transversale de la sortie d'air (15).

11. Dispositif d'inhalation (1) conformément à la revendication n°10,
**caractérisé par le fait que**
la structure de désagglomération (31) est constituée en forme de treillis, la structure de désagglomération (31) en forme de treillis étant formée de manière plane ou bombée, un sens de voûte et/ou un maillage pouvant être choisi en fonction de la poudre à inhaler au moyen du dispositif d'inhalation (1).

12. Utilisation d'un dispositif d'inhalation (1) conformément au moins à l'une des revendications n°1 à n°11 pour la perforation d'un récipient (2) avec une substance inhalable et pour la mise à disposition de la substance à inhaler.

13. Kit d'inhalation comprenant un dispositif d'inhalation (1) et au moins un récipient (2) avec une substance inhalable,
**caractérisé par le fait que**
le dispositif d'inhalation (1) est un dispositif d'inhalation (1) conformément au moins à l'une des revendications n°1 à n°11.

14. Kit d'inhalation conformément à la revendication n°13,
**caractérisé par le fait que**
le récipient (2) présente un rétrécissement (24) qui est disposé entre au moins un conduit d'amenée d'air (14', 14") et au moins un conduit d'échappement d'air (15') lors de la disposition du récipient (2) dans la cavité (17) du dispositif d'inhalation (1).
